# FASCICULE DE BREVET EUROPEEN

(11) **EP 0 654 458 B1**
(45) Date de publication et mention de la délivrance du brevet: **14.05.1997**
(21) Numéro de dépôt: 94420320.7
(22) Date de dépôt: 16.11.1994
(51) Int. Cl.: C07C 5/22, B01J 27/22

(54) **Procédé d'isomérisation d'hydrocarbures linéaires contenant au moins 7 atomes de carbone à l'aide de catalyseurs à base d'oxycarbure de molybdène**
Verfahren zur Isomerisierung von linearen Kohlenwasserstoffen mit wenigstens 7 Kohlenstoffatomen unter Verwendung von auf Molybdanoxycarbid enthaltenden Katalysatoren
Process for the isomerisation of linear hydrocarbons with at least 7 carbon atoms using catalysts based on molybdenum oxycarbide

(30) Priorité: 18.11.1993 FR 9314199
(43) Date de publication de la demande: 24.05.1995
(73) Titulaire: PECHINEY RECHERCHE (Groupement d'Intérêt Economique géré par l'ordonnance du 23 Septembre 1967), 92400 Courbevoie (FR)
(72) Inventeur: Ledoux, Marc J., F-67000 Strasbourg (FR); Guille, Jean-Louis, F-67000 Strasbourg (FR); Pham Huu, Cuong, F-67700 Saverne (FR); Blekkan, Edd, N-7079 Flataasen (NO); Peschiera, Eric, F-77210 Avon (FR)
(74) Mandataire: Mougeot, Jean-Claude

(56) Documents cités:
- EP-A- 0 396 475
- EP-A- 0 440 569
- EP-A- 0 474 570
- EP-A- 0 511 919
- EP-A- 0 534 867
- JOURNAL OF CATALYSIS, vol.143, no.1, Septembre 1993, DULUTH, MN US pages 249 - 261 CUONG PHAM-HUU ET AL 'Reactions of 2- and 3-Methylpentane, Methylcyclopentane, Cyclopentane, and Cyclohexane on Activated Mo2C'

## Description

### DOMAINE TECHNIQUE DE L'INVENTION

L'invention concerne le domaine des catalyseurs pour l'isomérisation des hydrocarbures linéaires en hydrocarbures ramifiés, présentant de ce fait un indice d'octane amélioré.

Ces réactions d'isomérisation ont un intérêt industriel considérable. En effet, le fonctionnement des moteurs à explosion demande des carburants à indice d'octane élevé. Cet indice élevé était obtenu par addition à l'essence de plomb tétraéthyle. Pour diminuer la pollution par le plomb, le plomb tétraéthyle est remplacé par d'autres produits à indice d'octane élevé, soit des aromatiques, néfastes aussi pour l'environnement, soit des éthers tertiaires. Ces produits entrent dans la composition des essences sans plomb.

A titre d'illustration, l'indice d'octane de l'isooctane (diméthyl-2,2 méthyl-3 pentane) est, par définition, de 100 et celui du n-heptane est de 0. Une coupe pétrolière naphta C₅-C₉ a un indice d'octane de 70 qui passe après réformage à 91 environ et après addition de plomb tétraéthyle à 94. Le toluène pur a un indice de 97.

La suppression dans les carburants du plomb tétraéthyle et des composés aromatiques donne un très grand intérêt à la recherche de procédés d'isomérisation permettant d'obtenir à partir d'hydrocarbures aliphatiques linéaires des isomères ramifiés dont l'indice d'octane est supérieur à celui de leurs homologues linéaires.

### ART ANTERIEUR

Une telle réaction d'isomérisation est bien connue des spécialistes: elle se pratique en faisant passer sur un catalyseur approprié l'hydrocarbure à isomériser dilué dans de l'hydrogène à une température voisine de 350°C. On recueille à la sortie du réacteur un mélange de différents isomères plus ou moins ramifiés, de produits craqués contenant donc moins d'atomes de carbone que l'hydrocarbure de départ et de produits cycliques. L'efficacité de la réaction se juge d'après le pourcentage de molécules de l'hydrocarbure de départ transformées en hydrocarbures ramifiés, les produits craqués ou cycliques étant considérés comme indésirables.

Les catalyseurs usuellement utilisés sont de type bifonctionnel: une fonction déshydrogénante-hydrogénante assurée par des métaux précieux: platine-rhénium ou platine-iridium et une fonction isomérisante acide assurée par le support, en général de l'alumine gamma dopée avec du chlore. Ensuite sont apparus d'autres catalyseurs composés de platine supporté par une zéolithe.
Plus récemment enfin, une nouvelle classe de catalyseurs a été trouvée, basée sur des espèces chimiques tout-à-fait différentes puisqu'il s'agit de catalyseurs à base de carbures de métaux lourds. De tels catalyseurs ainsi que leurs procédés de préparation sont décrits dans les demandes de brevets suivantes:

EP-A- 0 396 475 (PECHINEY ELECTROMETALLURGIE): Obtention de carbures de métaux lourds à surface spécifique élevée.
Cette demande décrit un procédé d'obtention de carbures de métaux lourds à surface spécifique élevée caractérisé en ce qu'on fait réagir un composé gazeux dudit métal avec un carbone réactif à surface spécifique d'au moins 200 m²/g à une température comprise entre 900°C et 1400°C, et les carbures ainsi obtenus.
Les exemples de cette demande comparent l'activité de ce nouveau type de catalyseurs, en particulier à base de carbures de tungstène et de molybdène avec un catalyseur classique, Al₂O₃ + 0,25 % de platine, pour l'isomérisation du méthylcyclopentane et du n-hexane.

EP-A- 0 440 569 (PECHINEY ELECTROMETALLURGIE): Procédé d'obtention de corps solides poreux à base de carbure réfractaire à l'aide de composés organiques et de métal ou métalloïde.
Cette demande décrit un procédé d'obtention de corps solides poreux de forme et porosité adaptées à la demande, en carbure à surface spécifique élevée, caractérisé en ce qu'on mélange un composé exclusivement organique polymérique et/ ou copolymérisable carbonisable et capable de donner un squelette solide carboné, avec une poudre de métal ou métalloïde ou un de ses composés réductible par le carbone, on met en forme le mélange, on réticule ou durcit le composé organique, on traite thermiquement pour carboniser entre 500 et 1000°C le composé organique, puis pour opérer la carburation.

EP-A-0 474 570 (GIE PECHINEY RECHERCHE): Procédé d'activation de la surface de carbures de métaux lourds à surface spécifique élevée en vue de réactions catalytiques.
Cette demande décrit un procédé d'activation de carbures de métaux lourds à surface spécifique élevée en vue de leur utilisation comme catalyseur de réactions chimiques ou pétrochimiques, consistant à traiter les carbures par oxydation ménagée sous courant de gaz oxydant à une température comprise entre 250 et 450°C en maintenant un palier de température d'au moins 3 heures puis en refroidissant jusqu'a température ambiante toujours sous courant oxydant. L'exemple 1 illustre la comparaison entre un catalyseur à base de carbure de molybdène activé selon le procédé décrit et un catalyseur classique à base Pt pour l'isomérisation du n-hexane.

EP-A-0 511 919 ((engobage) PECHINEY ELECTROMETALLURGIE): Système catalytique, notamment pour la postcombustion des gaz d'échappement et procédé pour le fabriquer.
Cette demande décrit un système catalytique constitué d'un support sur lequel le produit catalytiquement actif est déposé. Le support présente des propriétés mécaniques ou physiques intéressantes pour les conditions de fonctionnement requises, mais une surface spécifique médiocre. Le produit catalytiquement actif, un carbure métallique, est obtenu par engobage du support dans une suspension d'un composé réductible du métal dans une solution d'un composé organique, carbonisation de ce composé, réduction du composé métallique et carburation du métal. Le carbure ainsi obtenu présente une surface spécifique élevée.
De préférence, le support est constitué de carbure de silicium préparé par carbonisation d'une pâte contenant du silicium,du carbone et une résine organique. Dans les exemples, le carbure catalytiquement actif est un carbure de molybdène, de fer, de tungstène ou de vanadium.

EP-A- 0 534 867 (CIE PECHINEY RECHERCHE): Préparation de catalyseur à partir d'oxydes métalliques par réduction et carburation partielle par les gaz réactionnels.
Cette demande décrit un catalyseur pour les réactions chimiques et pétrochimiques constitué d'un oxyde d'un des métaux de transition, des terres rares ou d'un actinide, de molybdène par exemple, comportant en surface des carbures et oxycarbures.
Le procédé de fabrication consiste à faire passer sur l'oxyde le mélange gazeux réactionnel contenant des produits carbonés dont on veut opérer la transformation chimique catalytique à la température de cette réaction. Les produits carbonés présents dans le mélange provoquent une carburation progressive de la surface de l'oxyde et une augmentation progressive également de l'efficacité du catalyseur.
Le procédé s'applique en particulier à l'isomérisation des hydrocarbures pour lequel l'oxyde de molybdène MoO₃ constitue un catalyseur préféré. Les exemples décrivent l'isomérisation du n-hexane dilué dans de l'hydrogène.

### PROBLEME POSE. SOLUTION

Pour bien comprendre le problème posé aux chercheurs, il convient de définir un certain nombre de paramètres caractéristiques de la réaction d'isomérisation. Ces paramètres sont les suivants:

Taux de conversion C (en %): rapport du nombre de molécules d'hydrocarbure transformées soit par isomérisation soit par craquage au nombre de molécules passées sur le catalyseur.

Sélectivité totale S tot. (en %): rapport du nombre de molécules d'hydrocarbure isomérisées au nombre total de molécules transformées soit par isomérisation soit par craquage.

Sélectivité en produits acycliques S acy. (en %): rapport du nombre de molécules d'hydrocarbure isomérisées en produits acycliques au nombre total de molécules transformées soit par isomérisation soit par craquage.

Les catalyseurs classiques au platine de l'art antérieur permettent d'atteindre un taux de conversion et une sélectivité élevées lorsque la molécule à isomériser contient 6 atomes de carbone (n-hexane). Mais avec des hydrocarbures contenant plus de 6 atomes de carbone et cela dès le n-heptane, la sélectivité diminue fortement lorsque le taux de conversion augmente ("Isomérisation of C4-C7 paraffins on zeolithic catalysts" - M. Belloum et coll., Revue de l'Institut Français du Pétrole - 1991 vol. 41 p. 89-107).

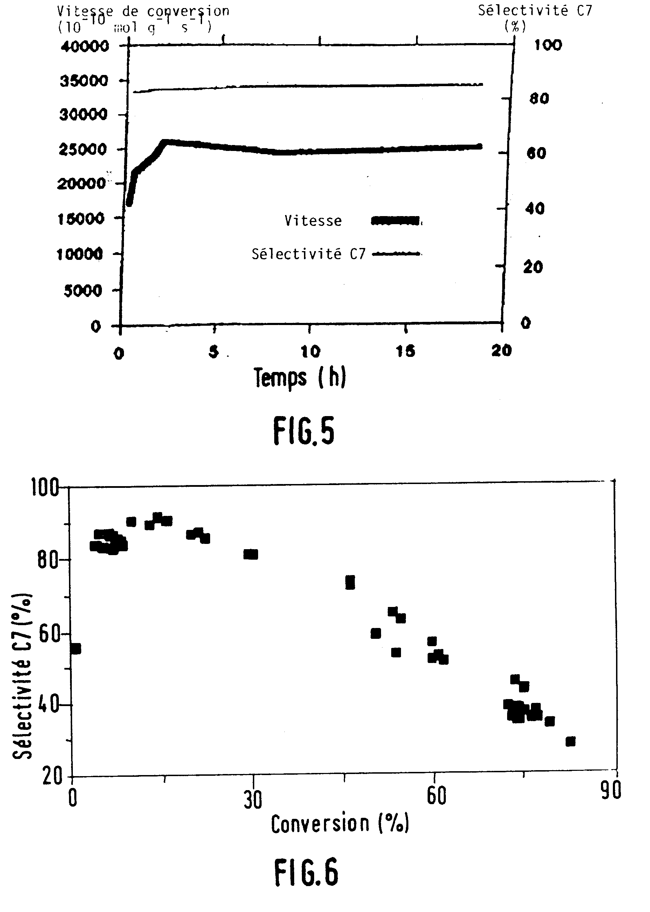

Ce phénomène est également illustré sur la figure 6, sur laquelle sont portés en abscisses le taux de conversion en régime stationnaire et en ordonnées la sélectivité en produits isomérisés correspondante.
Cette figure 6 demande quelques explications: tout d'abord les taux de conversion et les sélectivités indiqués sont ceux observés après quelques heures de fonctionnement, alors que le catalyseur a atteint son régime stationnaire et que ces valeurs se sont stabilisées. Ensuite, le taux de conversion dépend des conditions de la réaction: masse de catalyseur, débit de gaz, concentration de l'hydrocarbure dans le mélange réactionnel hydrocarbure-hydrogène, température, pression; il en est de même de la sélectivité.
On observe cependant une forte corrélation négative entre sélectivité et conversion, corrélation qui, en langage courant signifie que plus on transforme de molécules (ou augmente le taux de conversion), plus le pourcentage des produits ramifiés souhaités diminue. Cette diminution est, à l'évidence, un obstacle sérieux à l'utilisation des hydrocarbures supérieurs à C₆ comme source d'hydrocarbures ramifiés. En outre, avec de telles molécules plus riches en carbone, le craquage entraîne la formation de carbone libre qui colmate la surface active des catalyseurs et les rend progressivement inefficaces.

Actuellement, les hydrocarbures issus des coupes C₇-C₈ sont transformés par le réformage catalytique pour former des produits aromatiques utilisés comme ajouts dans les essences pour augmenter leur indice d'octane. Mais les nouvelles législations imposent une forte diminution des proportions d'aromatiques dans les essences, ainsi que cela a été indiqué plus haut.

Ainsi, si l'addition d'aromatiques a pu constituer dans le passé une solution, elle n'est maintenant plus possible et un procédé performant doit être trouvé pour obtenir des produits isomérisés à partir des hydrocarbures à longue chaîne. Un tel procédé n'existe pas avec les procédés catalytiques courants qui ont soit une sélectivité en isomères branchés insuffisante, soit un rendement de conversion (produit du taux de conversion par la sélectivité) également insuffisant pour en faire un procédé industriel économique.

Il est donc intéressant de trouver d'autres types de catalyseurs capables d'isomériser les hydrocarbures des coupes > C₆ avec des taux de conversion et des sélectivités en isomères aliphatiques mono ou multibranchés élevés et avec un minimum de produits aromatiques et de carbone de craquage.

Les inventeurs ont découvert que les catalyseurs à base de carbures et d'oxycarbures de molybdène préparés selon l'une ou l'autre des méthodes ci-dessus indiquées appliqués à l'isomérisation des carbures saturés contenant au moins 7 atomes de carbone et en particulier du n-heptane présentent cette propriété de sélectivité remarquable.

### OBJET DE L'INVENTION.

L'invention a pour objet un procédé d'isomérisation d'hydrocarbures linéaires comprenant au moins 7 (c'est-à-dire plus de 6) atomes de carbone par utilisation de catalyseurs à base de carbure et d'oxycarbure de molybdène.

### DESCRIPTION SOMMAIRE DES FIGURES

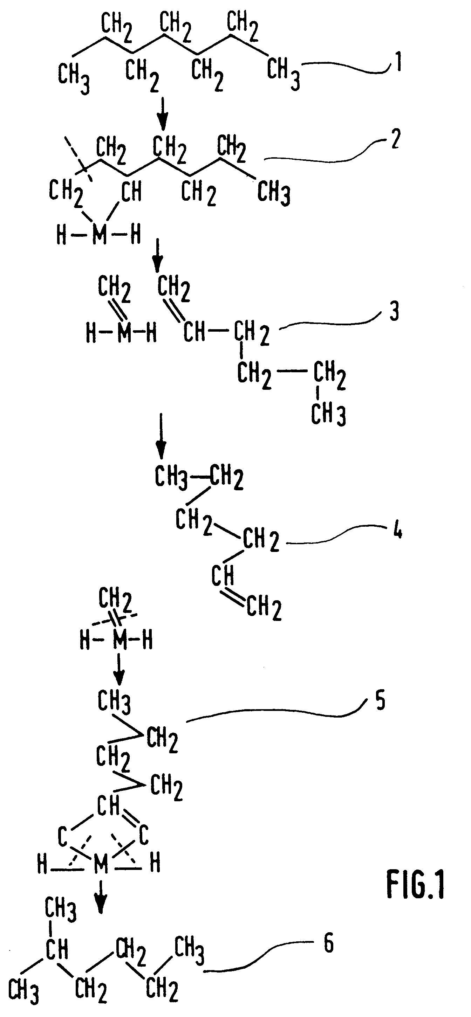

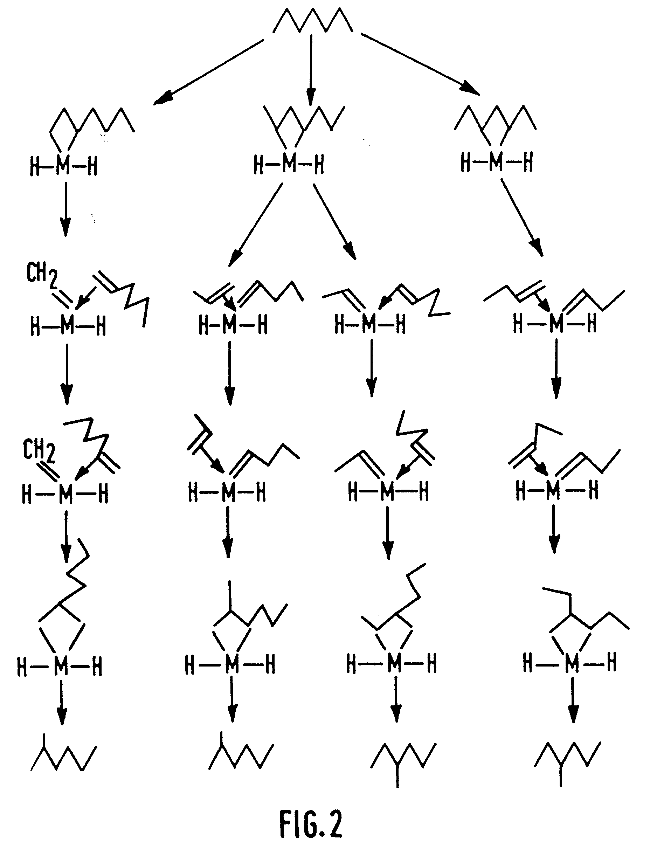

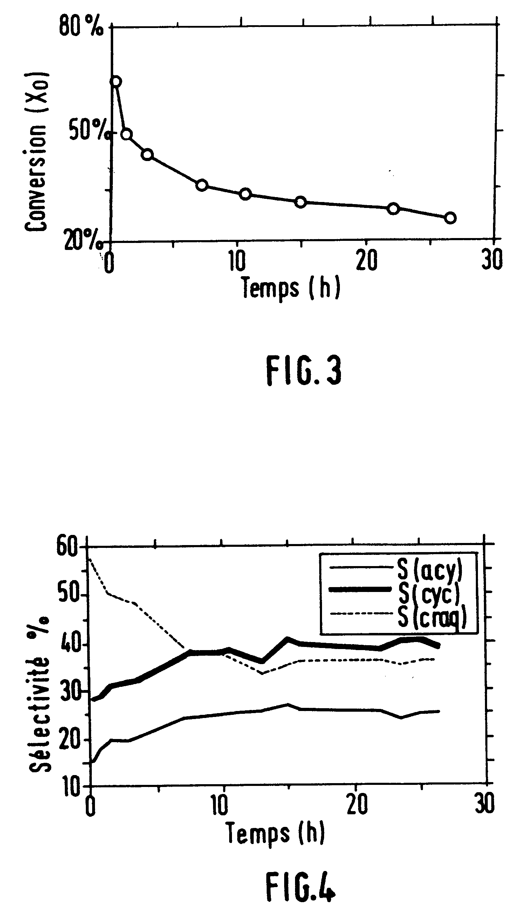

La figure 1 représente les étapes du mécanisme d'action d'un catalyseur à base d'oxycarbure de molybdène conduisant du n-heptane au méthyl-2-hexane.

La figure 2 représente en outre des mécanismes similaires conduisant aux deux isomères hexanes ramifiés, le méthyl-2-hexane et le méthyl-3-hexane.

La figure 3 représente en fonction du temps et pour un catalyseur de l'art antérieur, platine sur alumine, le taux de conversion (pourcentage de molécules d'heptane ayant réagi c'est-à-dire isomérisées ou craquées), la figure 4 les sélectivités en produits de craquage, en produits cycliques et en produits acycliques.

La figure 5 représente également en fonction du temps et pour un catalyseur de l'art antérieur à base de platine sur zéolithe, favorable à l'isomérisation, la vitesse de conversion (pourcentage de molécules de n-heptane ayant réagi par unité de temps et de poids de catalyseur employé, calculé sur la base d'une réaction du premier ordre par rapport à C7); cette vitesse est proportionnelle au taux de conversion et correspond ici à un bas taux de conversion inférieur à 20%

La figure 6 représente les valeurs des sélectivités en fonction des taux de conversion en régime d'équilibre relevées au cours de divers essais où l'on a fait varier les paramètres de la réaction d'isomérisation : température, pression, concentration du mélange en n-heptane, débit, pour un catalyseur selon l'art antérieur : Pt-zéolithe.

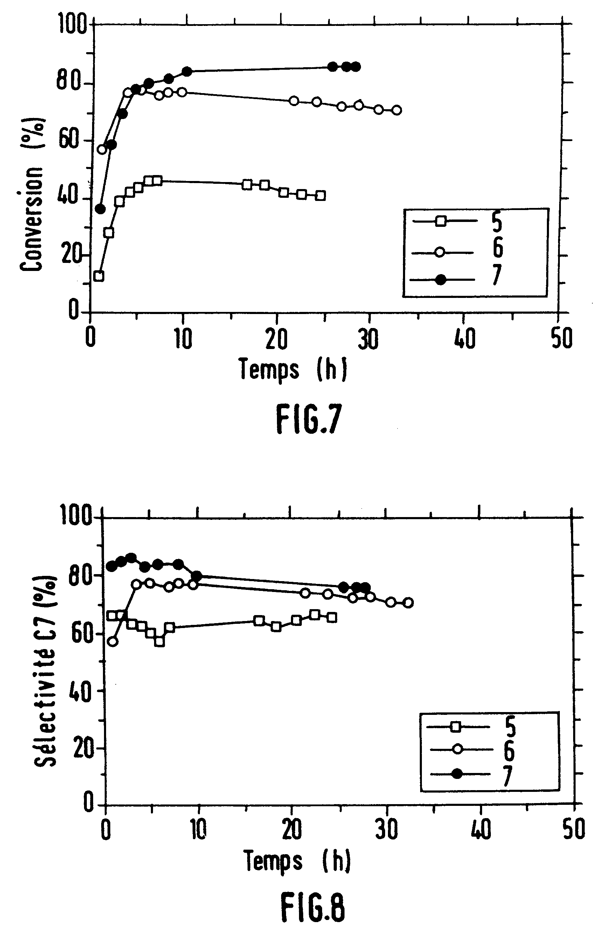

La figure 7 représente en fonction du temps le taux de conversion (pourcentage de molécules d'heptane ayant réagi c'est-à-dire isomérisées ou craquées), la figure 8 la sélectivité (pourcentage des molécules isomérisées par rapport aux molécules ayant réagi) pour un catalyseur selon l'invention à diverses valeurs de la pression totale.

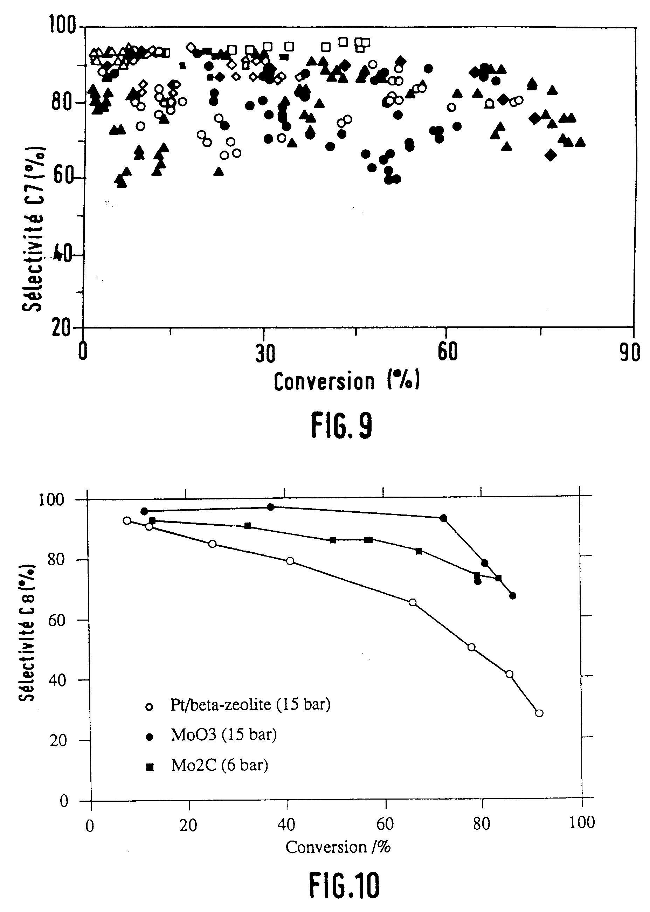

La figure 9 représente les valeurs des taux de conversion et des sélectivités en régime d'équilibre relevées au cours de divers essais où l'on a fait varier les paramètres de la réaction d'isomérisation: température, pression, concentration du mélange en n-heptane, débit, pour un catalyseur selon l'invention.

La figure 1O donne la sélectivité en fonction du taux de conversion lors de l'isomérisation du n-octane effectuée en présence de catalyseurs à base d'oxycarbure de molybdène selon l'invention ou en présence de catalyseur Pt-zéolithe selon l'art antérieur.

### DESCRIPTION DE L'INVENTION.

Le procédé objet de l'invention se caractérise par la combinaison d'un catalyseur spécifique et d'un mélange réactionnel dans des conditions opératoires particulières.

Le catalyseur utilisé comprend des composés de molybdène dont au moins la surface active au contact avec le mélange gazeux à traiter est constituée de carbure de molybdène partiellement oxydé sous forme d'un ou plusieurs oxycarbures. Cette phase superficielle d'oxycarbure a été mise en évidence par différentes techniques physicochimiques telles la diffraction des rayons X, la réduction par température programmée, la spectroscopie des photoélectrons induits par les rayons X, la microscopie électronique à balayage, la microscopie électronique par transmission et l'analyse par dispersion d'énergie des rayons X.

Pour obtenir cet oxycarbure superficiel, plusieurs voies sont possibles.

### a) A partir de carbure de molybdène Mo₂C.

On peut tout d'abord fabriquer du carbure de molybdène à grande surface spécifique:
- soit en faisant réagir un composé volatil du molybdène, MoO₃ sur du carbone à grande surface spécifique, selon l'enseignement de la demande de brevet EP-A- 0 396 475; il n'est pas nécessaire de transformer la totalité du carbone en Mo₂C, on peut se contenter d'une couche superficielle de Mo₂C et d'un coeur en carbone ou de préférence en un autre carbure, par exemple en SiC servant de support;
- soit en carbonisant une pâte composée d'un mélange d'une résine organique, de poudre de molybdène ou d'un composé réductible du molybdène et en chauffant ensuite le mélange carbonisé à une température suffisante pour réduire le composé de molybdène et carburer le molybdène obtenu, selon l'enseignement de la demande de brevet EP-A- 0 440 569. Comme dans la méthode précédente, on peut obtenir une couche active de carbure de molybdène sur un support en immergeant ce support, en carbure de silicium par exemple, dans une suspension de Mo ou d'un composé réductible de Mo dans un liquide organique carbonisable. La suspension imprégnant le support est ensuite carbonisée, le composé de Mo réduit et le molybdène obtenu carburé par chauffage, selon l'enseignement de la demande de brevet EP-A-0 511 919.

Ce carbure de molybdène préparé par l'une ou l'autre méthode doit être alors activé afin que se développent en surface les oxycarbures responsables de l'activité catalytique. Cette activation s'opère, selon l'enseignement de la demande de brevet EP-A-0 474 570, par traitement du carbure par oxydation ménagée sous courant de gaz oxydant à une température comprise entre 250 et 450°C.
Ce traitement d'activation doit être suivi d'un traitement d'équilibrage consistant à faire passer sur le catalyseur activé, à une température comprise entre 250 et 450°C et de préférence voisine de 350°C ou bien un mélange de n-hexane et d'hydrogène ou bien le mélange réactionnel lui-même composé d'un mélange d'hydrogène et d'hydrocarbure à plus de 6 atomes de carbone.

### b) A partir de molybdène métal ou d'oxyde de molybdène MoO₃.

On peut aussi partir de molybdène métal ou d'oxyde de molybdène MoO₃. Si l'on part du Mo métallique, la première étape consiste à oxyder superficiellement la poudre ou les grains de molybdène en oxyde MoO₃. A partir soit de Mo métallique recouvert d'une couche de MoO₃, soit de MoO₃ pur, il est possible, selon l'enseignement de la demande de brevet EP-A- 0 534 867 de développer en surface du MoO₃ une phase oxycarbure. Il suffit pour cela de faire passer à une température de 250 à 450°C sur le MoO₃ superficiel ou bien un mélange de n-hexane et d'hydrogène ou bien le mélange réactionnel lui-même composé d'un mélange d'hydrogène et d'hydrocarbure à plus de 6 atomes de carbone. Bien entendu, le traitement d'activation par un gaz oxydant est inutile puisque l'on part d'un oxyde et non pas d'un carbure.

En plus des catalyseurs cités ci-dessus, il convient de mentionner spécialement un catalyseur composé (i) d'un support en un produit présentant une interaction faible avec l'oxyde de molybdène MoO₃ et (ii) d'une surface ou couche active en oxyde de molybdène MoO₃. Les supports présentant cette interaction faible avec MoO₃ sont en particulier TiO₂, SiO₂, ZrO₂, et surtout le carbure de silicium SiC.
Ces catalyseurs supportés sont fabriqués de la façon suivante: Le support poreux est imprégné d'une solution aqueuse d'heptamolybdate d'ammonium en quantité telle que la teneur, mesurée en molybdène métal soit comprise entre 5 et 16 % du poids du support. La solution est versée goutte à goutte sur le support. Après séchage pendant de 8 à 20 heures à 100-150°C, le catalyseur est calciné sous air à une température comprise entre 400 et 600°C environ pour calciner le molybdate et obtenir MoO₃.

Le mélange réactionnel auquel sont appliqués ces catalyseurs, obtenus par l'une des voies décrites ci-dessus, constitue un élément essentiel de l'invention. En effet, alors qu'avec les catalyseurs à base de métaux précieux, l'isomérisation des hydrocarbures à plus de 6 atomes de carbone et en particulier du n-heptane se caractérise par une diminution sensible de la sélectivité lorsque le taux de conversion augmente et par le dépôt de carbone provenant du craquage, avec les catalyseurs à base d'oxycarbure de molybdène, elle conduit à une forte proportion d'hydrocarbures aliphatiques ramifiés et à une faible proportion de composés cycliques et de carbone, cette sélectivité élevée étant pratiquement constante quel que soit le taux de conversion.
Le mélange réactionnel de l'invention comprend un ou plusieurs hydrocarbures linéaires ayant un nombre d'atomes de carbone d'au moins 7 dilués dans de l'hydrogène. Il peut contenir également des hydrocarbures linéaires ayant un nombre d'atomes de carbone inférieur à 7.

Les conditions opératoires préférées de la réaction d'isomérisation sont les suivantes:
- température comprise entre 250 et 450°C
- concentration en volume du ou des hydrocarbures dans l'hydrogène comprise entre 1 et 70%
- pression totale du mélange réactionnel de 100 à 2000 kPa.

Cette supériorité des catalyseurs à base d'oxycarbure de Mo sur les catalyseurs Pt-alumine ou Pt-zéolithe s'explique par un mécanisme de catalyse différent.

Ainsi que cela a été expliqué plus haut, les catalyseurs usuellement utilisés sont de type bifonctionnel: une fonction déshydrogénante-hydrogénante assurée par des métaux précieux: platine-rhénium ou platine-iridium et une fonction isomérisante acide assurée par le support, en général de l'alumine gamma dopée avec du chlore ou des zéolithes acides. Ainsi ce n'est pas seulement le métal précieux qui participe à l'activité catalytique, mais également le support en alumine ou en zéolithe. Le mécanisme de la réaction s'explique de la façon suivante: dans une première étape, il y a déshydrogénation de la molécule d'hydrocarbure, catalysée par le platine; dans une deuxième étape, la molécule déshydrogénée est isomérisée, réaction catalysée par la fonction acide de l'alumine ou de la zéolithe et enfin dans une troisième étape l'isomère obtenu est réhydrogéné avec formation d'un isomère du produit de départ.

Dans le cas des catalyseurs à base d'oxycarbure de molybdène, le mécanisme proposé est tout-à-fait différent. Il a été décrit pour les C6, dans l'article "Reactions of 2- and 3-méthylpentane, méthylcyclopentane, cyclopentane, and cyclohexane on actived Mo₂C" de Cuong Pham-Huu, Marc-J. Ledoux and Jean Guille, publié dans "Journal of Catalysis, 143, 249-261". Il est représenté schématiquement pour le cas des C7 sur les figures 1 et 2.
La figure 1, plus détaillée, montre les étapes conduisant du n-heptane au méthyl-2-hexane. La figure 2 représente en outre d'autres possibilités, basées sur un mécanisme similaire conduisant à deux isomères hexanes ramifiés, le méthyl-2-hexane et le méthyl-3-hexane.

La première étape, (figure 1), consiste en tous les cas, à partir de l'heptane normal (1), en la formation d'un composé métallocyclique (2) composé de trois atomes de carbone et d'un atome de molybdène. Les 3 atomes de carbone peuvent être les carbones 1, 2, 3 (figures 1 et 2A) ou les carbones 2, 3, 4 (figure 2B) ou enfin les carbones 3, 4, 5 (figure 2C).
Dans la deuxième étape, figure 1, la liaison entre les carbones 1 et 2 est rompue et l'on obtient les deux composés (3): un composé métallique muni d'un radical méthylidène et de l'hexène 1-2.
Le retournement de la molécule d'hexène conduit dans une troisième étape à la configuration (4).
Dans une quatrième étape, l'ouverture des doubles liaisons conduit de nouveau à un composé métallocyclique (5) isomère du composé (2).
Enfin, au cours de la cinquième étape, ce composé métallocyclique est décomposé en donnant du méthyl-2-hexane.

Les figures 2B et 2C illustrent des mécanismes analogues qui entrent en compétition avec celui décrit ci-dessus.

Il est à remarquer que le procédé d'isomérisation des hydrocarbures linéaires contenant au moins 7 atomes de carbone selon l'invention se révèle être une application des catalyseurs à base de carbure et d'oxycarbure de molybdène particulièrement intéressante et d'un grand intérêt.

En effet ledit procédé de l'invention permet d'obtenir des rendements d'isomérisation en produits acycliques très élevés; ce rendement élevé vient de ce que le procédé peut être conduit de façon à avoir un taux de conversion élevé (dépassant aisément 50%, pour aller jusqu'à l'équilibre thermodynamique, c'est-à-dire de 80 à 95% selon les molécules obtenues), sans que soit affectée significativement la sélectivité qui reste à un niveau également très élevé. Autrement dit la sélectivité est indépendante du taux de conversion et cela, quel que soit le nombre de carbone de l'hydrocarbure à isomériser.

En comparaison les procédés d'isomérisation utilisant des catalyseurs bifonctionnels (Pt ou métaux précieux déposés sur support d'alumine ou zéolithe) ne permettent pas un tel résultat.
Ainsi, il faut noter que le mécanisme de la catalyse bi-fonctionnelle ne s'applique pas à tous les hydrocarbures, notamment ceux ayant plus de 6 atomes de carbone, alors qu'au contraire le mécanisme de l'isomérisation en présence d'oxycarbure marche aussi bien pour les hydrocarbures légers ayant au plus 6 atomes de carbures que pour les hydrocarbures à longues chaînes ayant 7 et plus atomes de carbone.

En conséquence il va être possible de traiter industriellement non seulement les fractions les plus lourdes des coupes naphta (C8 à C10 par exemple) pour les transformer en isomères acycliques à fort indice d'octane, mais aussi les coupes beaucoup plus lourdes, notamment celles entrant dans la composition des carburants pour moteurs Diesel (C10 et au-delà) ou encore celles donnant les huiles et lubrifiants (C17 et au-delà) pour lesquelles l'isomérisation permet un déparafinage et une diminution de la viscosité (pour les huiles).

### EXEMPLES

Les exemples suivants permettront d'illustrer le procédé de l'invention appliqué à différents hydrocarbures.

Les résultats sont présentés sous forme de tableaux indiquant, en fonction du temps écoulé t depuis le début de la réaction, c'est-à-dire depuis le début du passage du produit à isomériser sur le catalyseur, la valeur d'un certain nombre de paramètres définis comme suit:

Conversion C (en %): rapport du nombre de molécules de n-heptane transformées soit par isomérisation soit par craquage au nombre de molécules passées sur le catalyseur.

Sélectivité totale S tot. (en %): rapport du nombre de molécules de n-heptane isomérisées au nombre total de molécules transformées soit par isomérisation soit par craquage.

Sélectivité en produits acycliques S acy. (en %): rapport du nombre de molécules de n-heptane isomérisées en produits acycliques au nombre total de molécules transformées soit par isomérisation soit par craquage.

Sélectivité en produits craqués S crq. (en %): rapport du nombre de molécules de n-heptane craquées au nombre total de molécules transformées soit par isomérisation soit par craquage.

On a bien évidemment la relation: Stot + Scrq = 100 %

Les exemples 1 à 4 concernent l'isomérisation du n-heptane selon l'art antérieur en présence d'un catalyseur bifonctionnel à base de Pt et d'alumine gamma (exemple 1) ou de Pt et zéolithe (exemple 2), selon la présente invention en présence d'un catalyseur à base d'oxycarbure de molybdène (exemple 3) et une comparaison des courbes de sélectivité en fonction du taux de conversion avec un catalyseur selon l'invention et un catalyseur Pt-zéolithe selon l'art antérieur.

L'exemple 5 est une comparaison de ces mêmes courbes pour le n-octane, tandis que les exemples 6 et 7 concernent l'isomérisation des carbures en C10 et C20 et l'exemple 8 l'isomérisation du n-heptane en présence d'un catalyseur à base d'oxycarbure de molybdène déposé sur un support de carbure.

### Exemple 1 comparatif selon l'art antérieur.

### Catalyseur à base de platine supporté sur alumine gamma

Les espèces chimiques issues de la réaction catalytique concernant le n-heptane sont les suivantes:
1°) Produits isomérisés:
   - Acycliques
      - DMP = ensemble des diméthyl-2.2 pentane + diméthyl-2.3 pentane + diméthyl-2.4 pentane;
      - M2H, M3H = méthyl 2- et méthyl 3-hexane;
      - E3P = éthyl 3-pentane;
      - Multibr. = divers heptanes multibranchés.
   - Cycliques
      - MeCyH = méthylcyclohexane;
      - EcyP = éthylcyclopentane;
      - Toluène.
      - Cyc = ensemble des composés cycliques en C5.
2°) Produits craqués:
   - C6 + C1 = 1 mole hexane + 1 mole méthane;
   - C5 + C2 = 1 mole pentane + 1 mole éthane;
   - C4 + C3 = 1 mole butane + 1 mole propane;
   - 2C3 +C1 = 2 moles propane + 1 mole méthane;
   - 3C2 + C1 = 3 moles éthane + 1 mole méthane;
   - 7C1 = 7 moles méthane.

Le catalyseur est préparé à partir d'alumine gamma ayant les caractéristiques suivantes:
- volume poreux: 0,62 cm³/g;
- granulométrie comprise entre 0,250 µm et 0,425 µm;
- surface spécifique: 217 m²/g.

Cette alumine est imprégnée d'une solution de H₂PtCl₆.xH₂O. Après imprégnation, le catalyseur est séché à l'air à la température ambiante pendant 8 heures, puis calciné à 400°C pendant 2 heures. La réduction de l'acide chloroplatinique a lieu sous flux d'hydrogène à 400°C pendant 2 heures. On obtient ainsi un catalyseur comprenant, en poids, 1,3 % de platine. Puis la température est abaissée à 350°C et l'hydrogène est remplacé par un mélange de n-heptane et d'hydrogène sous pression totale de 1013 hPa = 1 atm, la pression partielle du n-heptane étant de 16,15 hPa. Dans cet exemple, le débit de mélange gazeux est de 40 cm³/min et la masse de catalyseur de 0,0245g.

Les taux de conversion et les sélectivités figurent dans le tableau 1 ci-après.

**TABLEAU 1**

| | | | | | | |
|---|---|---|---|---|---|---|
| Temps(heures) | 0,25 | 2,5 | 8 | 10,5 | 15 | 25 |
| Conv. C(%) | 65 | 44 | 34 | 33 | 31 | 28 |
| Sél. tot(%) | 43 | 51 | 62 | 63 | 65 | 64 |
| Sél.acy(%) | 15 | 19,5 | 24,3 | 25 | 26 | 25 |
| Sél.craq(%) | 57 | 49 | 38 | 37 | 35 | 36 |

Ces résultats appellent les commentaires suivants:

Le taux de conversion du catalyseur diminue fortement pendant les 8 premières heures de la réaction, passant de 65 % à 34 %, puis plus faiblement ensuite.

La sélectivité en molécules craquées, élevée au départ (57 %), décroît pour se fixer à une valeur voisine de 36 %. Corrélativement, la sélectivité totale en produits croît de 43 % à 64 %. Mais les isomères obtenus sont caractérisés par une forte proportion d'hydrocarbures cycliques, méthylcyclohexane, éthylcyclopentane et toluène. Cette proportion d'isomères cycliques croît plus vite que la proportion d'isomères acycliques puisque si la sélectivité totale augmente en gros de 20 points, la sélectivité en isomères acycliques n'augmente, elle, que de 10 points pour s'établir en régime stationnaire, à une valeur faible de 25 %.

Les résultats sont illustrés sur les figures 3 et 4.
La figure 3 représente en fonction du temps l'évolution du taux de conversion et la figure 4 l'évolution, en fonction du temps, des sélectivités en produits de craquage, en produits cycliques et en produits acycliques.

### Exemple 2

### Isomérisation du n-heptane en présence d'un catalyseur à base de platine sur support de zéolithe selon l'art antérieur.

Le catalyseur comporte 0,8% Pt sur un support de béta-zéolithe.

Conversion correspondant pour différentes valeurs de la température et de la pression.

| | | | | | |
|---|---|---|---|---|---|
| Température K | 523 | 523 | 523 | 548 | 573 |
| Pression bar | 6,5 | 6,5 | 16,5 | 16,5 | 16,5 |
| Conversion % | 8 | 8 | 16 | 30 | 62 |
| Sélectivité en C7 | 84 | 85 | 90 | 81 | 52 |

Par ailleurs, la figure 5 donne, en fonction du temps, la vitesse d'isomérisation qui est obtenue à partir du taux de conversion par unité de temps et par unité de poids du catalyseur mis en oeuvre, sur la base d'une réaction du 1er ordre par rapport à C7. Cette vitesse est proportionnelle au taux de conversion et correspond dans le cas présent à un bas taux de conversion toujours inférieur à 20%.
Quant à la figure 6, elle donne la sélectivité en fonction du taux de conversion.

Cet exemple permet de voir qu'on ne peut jamais avoir simultanément un fort taux de conversion et une forte sélectivité, donc un rendement de conversion (qui est le produit de ces deux valeurs) élevé.
Un tel catalyseur n'est donc pas exploitable industriellement pour isomériser des hydrocarbures linéaires ayant au moins 7 atomes de carbone.

### Exemple 3

### Isomérisation du n-heptane sur oxycarbure de molybdène préparé à partir de MoO₃. Influence de la pression totale du système.

On utilise le même réacteur que celui décrit dans l'exemple 1, mais les essais sont réalisés sous trois différentes pressions totales:
3 bars = 300 kPa essai 5
6 bars = 600 kPa essai 6
20 bars = 2000 kPa essai 7

Les autres conditions opératoires sont les suivantes:

| | essais 5 et 6 | essai 7 |
|---|---|---|
| Température | 350°C | 350°C |
| Masse de MoO3 | 0,3 g | 0,33 g |

| Débits | | |
|---|---|---|
| -en n-heptane liqu. | 0,02 cm3/min | 0,025 cm3/min |
| -en n-heptane gaz | 3,05 cm3/min | 3,82 cm3/min (CNTP) |
| Débit d'hydrogène | 120 cm3/min | 150 cm3/min (CNTP) |
| Rapport H2/heptane | 39 | 39 |

Les résultats obtenus sont représentés sur les figures 7 et 8. La figure 7 représente en fonction du temps le taux de conversion (pourcentage de molécules d'heptane ayant réagi c'est-à-dire isomérisées ou craquées), la figure 8 la sélectivité (pourcentage des molécules isomérisées par rapport aux molécules ayant réagi).

On constate que le taux de conversion atteint un état stationnaire pour l'essai 5 à environ 41 % au bout de 20 heures, pour l'essai 6 à environ 70 % au bout de 25 heures, enfin pour l'essai 7 à environ 85 % au bout de 20 heures. La sélectivité à l'état stationnaire est de 62 % pour l'essai 5, de 75 % pour l'essai 6 et de 78 % pour l'essai 7.

La comparaison avec l'exemple 1 est dans ce cas encore plus démonstrative.

| | Taux de conversion | Sélectivité |
|---|---|---|
| Exemple 1 | 34 % | 25 % |
| Essai 5 | 41 % | 62 % |
| Essai 6 | 70 % | 75 % |
| Essai 7 | 85 % | 78 % |

### Exemple 4

### Synthèse des résultats.

Sur la figure 9 on reporté les valeurs des taux de conversion et des sélectivités en régime d'équilibre relevées au cours de divers essais où l'on a fait varier les différents paramètres de la réaction d'isomérisation: température, pression, concentration du mélange en n-heptane, débit. Les taux de conversion sont portés en abscisses et les sélectivités en ordonnées. En dépit d'une certaine dispersion, on constate qu'il n'existe pratiquement aucun point présentant un taux de conversion inférieur à 60% et que par contre des points avec un taux de conversion voisin de 75% ont une sélectivité voisine de 90%. De plus le nuage de points a son grand axe parallèle à l'axe des abscisses, ce qui signifie que la sélectivité ne dépend pas du taux de conversion.

Cette figure peut être avantageusement comparée à la figure 6 (exemple 2) relative à l'isomérisation du n-heptane en présence de Pt-zéolithe où la corrélation sélectivité-taux de conversion est nettement négative et où pour un taux de 75% la sélectivité ne dépasse pas environ 40%.

### Exemple 5

Les courbes de sélectivité en fonction du taux de conversion ont été tracées dans le cas de l'isomérisation du n-octane (figure 10). Les catalyseurs utilisés sont le Pt-zéolithe de l'art antérieur (comme dans l'exemple 4), et l'oxycarbure de molybdène obtenu selon deux modes: à partir de MoO₃ pour donner un oxycarbure de 140 m²/g de surface spécifique comme décrit plus haut, ou à partir de Mo₂C à grande surface spécifique (125 m²/g) lui-même obtenu par réaction de MoO₃ gazeux sur un carbone réactif à grande surface spécifique (comme décrit dans la demande EP 0396475 vue plus haut), le carbure étant ensuite traité à l'oxygène (sous air, à 350°C pendant 14 h).

Les réactions catalytiques ont été effectuées sous pression. Dans la figure 10 les désignations MoO₃ et Mo₂C représentent les oxycarbures correspondants.

On voit là encore que pour des taux de conversion très élevés, la sélectivité qui est essentiellement acyclique (les produits cycliques ne dépassant pas en effet environ 2%) varie peu pour les réaction en présence d'oxycarbures de molybdène, alors que ce n'est pas le cas pour le catalyseur classique.

Ceci est d'un grand intérêt industriel, car on voit que le rendement de conversion est élevé alors qu'avec le catalyseur Pt-zéolithe il faut se contenter d'un taux de conversion qui ne dépasse pas 40%.

### Exemple 6

Cet exemple concerne l'isomérisation des hydrocarbures en C10 et C20.
Elle a été effectuée en présence d'oxycarbure obtenu à partir de MoO₃, comme dans l'exemple précédent, et sous une pression de 7 bars.
Les résultats sont donnés dans le Tableau 2 ci-dessous, où figurent le taux de conversion et la sélectivité correspondante.

**TABLEAU 2**

| | Conversion (%) | Sélectivité (%) |
|---|---|---|
| C10 | 40 | 94 |
| | 60 | 90 |
| C20 | 39 | 91 |
| | 62 | 89 |

On voit qu'on obtient à la fois un taux de conversion et une sélectivité élevés; par ailleurs il a été possible de noter que plus de la moitié des isomères obtenus étaient multi-branchés, c'est-à-dire possédant deux et plus atomes de carbone substitués, ce qui représente un avantage certain, la présence d'isomères cycliques demeurant toujours très faible.

Ces résultats sont assez inhabituels pour des hydrocarbures à longue chaîne de ce type.

### Exemple 7

Cet exemple concerne l'isomérisation du n-heptane en présence d'un catalyseur à base d'oxycarbure déposé sur un support en SiC à surface spécifique élevée de 127 m²/g.
Le support a été obtenu selon le procédé du brevet français 2621904 à partir de grains de carbone réactif de surface spécifique BET de 987 m²/g.
Le support a été imprégné par de l'heptamolybdate d'ammonium, calciné à 550°C pendant 2 heures puis traité ensuite directement par le gaz réactionnel contenant le n-heptane à 350°C sous 20 bars, le rapport en volume H2/n-heptane étant de 40, pour obtenir le catalyseur à base d'oxycarbure de molybdène à surface spécifique élevée.
Le traitement d'isomérisation a été poursuivi dans les mêmes conditions.
Le rapport pondéral oxycarbure de molybdène/support de SiC était de 15%.

Comme dans les exemples précédents, on a obtenu des valeurs élevées de la sélectivité (92%) à forte conversion (83%) qui de plus demeurent constantes au cours du temps.

## Revendications

1. Procédé d'isomérisation d'hydrocarbures linéaires comprenant au moins 7 atomes de carbone caractérisé en ce que l'on fait passer un mélange réactionnel, de préférence sous une pression totale de 100 à 2000 kPa, contenant le ou les hydrocarbures à isomériser et de l'hydrogène sur un catalyseur comprenant des composés de molybdène et dont au moins la surface est constituée de carbure de molybdène partiellement oxydé sous forme d'un ou plusieurs oxycarbures.

2. Procédé d'isomérisation selon la revendication 1 caractérisé en ce que le passage du mélange réactionnel sur le catalyseur se fait à une température comprise entre 250 et 450°C, à une concentration en volume du ou des hydrocarbures dans l'hydrogène comprise entre 1 et 70%.

3. Procédé d'isomérisation d'hydrocarbures selon l'une des revendications 1 ou 2, caractérisé en ce que le catalyseur est préparé en faisant réagir un composé volatil du molybdène, MoO₃ sur du carbone à grande surface spécifique puis en activant le carbure de molybdène formé par oxydation ménagée sous courant de gaz oxydant à une température comprise entre 250 et 450°C.

4. Procédé d'isomérisation d'hydrocarbures selon l'une des revendications 1 ou 2, caractérisé en ce que le catalyseur est préparé en carbonisant une pâte composée d'un mélange d'une résine organique, de poudre de molybdène ou d'un composé réductible du molybdène et en chauffant ensuite le mélange carbonisé à une température suffisante pour réduire le composé de molybdène et carburer le molybdène obtenu, puis en activant le carbure de molybdène formé par oxydation ménagée sous courant de gaz oxydant à une température comprise entre 250 et 450°C.

5. Procédé d'isomérisation d'hydrocarbures selon l'une des revendications 1 ou 2, caractérisé en ce que le catalyseur est obtenu en immergeant un support minéral dans une suspension de Mo ou d'un composé réductible de Mo dans un liquide organique carbonisable, en carbonisant la suspension imprégnant le support, en réduisant le composé de Mo, en carburant le molybdène obtenu, enfin en activant le carbure de molybdène formé par oxydation ménagée sous courant de gaz oxydant à une température comprise entre 250 et 450°C.

6. Procédé d'isomérisation d'hydrocarbures selon l'une des revendications 3 à 5, caractérisé en ce que le catalyseur est soumis, avant le passage du mélange réactionnel à isomériser à un traitement consistant à faire passer à une température comprise entre 250 et 450°C sur le catalyseur activé un mélange sous pression atmosphérique de n-hexane et d'hydrogène.

7. Procédé d'isomérisation d'hydrocarbures selon l'une des revendications 1 ou 2, caractérisé en ce que le catalyseur est obtenu en oxydant superficiellement du Mo métallique en oxyde MoO₃ et en ce que le MoO₃ superficiel est carburé partiellement par passage du mélange réactionnel lui-même contenant de l'hydrogène et le ou les hydrocarbures à une température comprise entre 250 et 450°C.

8. Procédé d'isomérisation d'hydrocarbures selon l'une des revendications 1 ou 2, caractérisé en ce que le catalyseur est obtenu à partir de MoO₃ que l'on carbure partiellement par passage du mélange réactionnel lui-même contenant de l'hydrogène et le ou les hydrocarbures à une température comprise entre 250 et 450°C.

9. Procédé d'isomérisation d'hydrocarbures selon l'une des revendications 7 ou 8, caractérisé en ce que, avant de faire passer le mélange réactionnel sur l'oxyde de molybdène MoO₃, cet oxyde est carburé partiellement par passage d'un mélange de n-hexane et d'hydrogène à une température comprise entre 250 et 450°C.

10. Procédé d'isomérisation d'hydrocarbures selon l'une des revendications 1 ou 2, caractérisé en ce que le catalyseur est obtenu à partir d'un support poreux en un produit présentant une interaction faible avec l'oxyde de molybdène MoO₃, recouvert d'une couche d'oxyde de molybdène MoO₃ qui est ensuite carburée partiellement par passage d'un mélange d'hydrocarbures et d'hydrogène à une température comprise entre 250 et 450°C.

11. Procédé d'isomérisation d'hydrocarbures selon la revendication 10, caractérisé en ce que le support présentant une interaction faible avec l'oxyde de molybdène appartient au groupe des oxydes TiO₂, SiO₂, ZrO₂.

12. Procédé d'isomérisation d'hydrocarbures selon la revendication 10, caractérisé en ce que le support présentant une interaction faible avec l'oxyde de molybdène est en carbure de silicium SiC.

13. Procédé d'isomérisation d'hydrocarbures selon l'une des revendications 10 à 12, caractérisé en ce que le support poreux est recouvert d'une couche de MoO₃ par imprégnation avec une solution aqueuse d'heptamolybdate d'ammonium en quantité telle que la teneur, mesurée en molybdène métal soit comprise entre 5 et 16 % du poids du support, séché pendant de 8 à 20 heures à une température comprise entre 100 et 150°C, puis calciné sous air à une température comprise entre 400 et 600°C pour transformer le molybdate en MoO₃.

14. Procédé d'isomérisation d'hydrocarbures selon l'une des revendications 10 à 13, caractérisé en ce que le mélange d'hydrocarbures et d'hydrogène est le mélange réactionnel lui-même, comprenant le ou les hydrocarbures ayant plus de 6 atomes de carbone et de l'hydrogène.

15. Procédé d'isomérisation d'hydrocarbures selon l'une des revendications 10 à 13, caractérisé en ce que le mélange d'hydrocarbure et d'hydrogène est dans un premier temps un mélange de n-hexane et d'hydrogène et dans un deuxième temps le mélange réactionnel, comprenant le ou les hydrocarbures ayant au moins 7 atomes de carbone et de l'hydrogène.

16. Procédé d'isomérisation d'hydrocarbures selon l'une des revendications 1 à 15, caractérisé en ce que l'hydrocarbure du mélange réactionnel ayant au moins 7 atomes de carbone est du n-heptane.

17. Procédé d'isomérisation d'hydrocarbures selon l'une des revendications 1 à 15, caractérisé en ce que le mélange réactionnel contient des hydrocarbures contenant de 8 à 10 atomes de carbone.

## Claims

1. A process for the isomerisation of straight hydrocarbons comprising at least 7 carbon atoms, characterised in that a reaction mixture which contains the hydrocarbon(s) to be isomerised and hydrogen is passed over, preferably under a total pressure from 100 to 2000 kPa, a catalyst comprising molybdenum compounds, at least the surface of which is made up of molybdenum carbide partially oxidised in the form of one or more oxycarbides.

2. An isomerisation process according to Claim 1, characterised in that the reaction mixture is passed over the catalyst at a temperature of between 250 and 450°C, at a concentration by volume of the hydrocarbon(s) in hydrogen of between 1 and 70%.

3. A process for the isomerisation of hydrocarbons according to one of Claims 1 and 2, characterised in that the catalyst is prepared by reacting a volatile compound of molybdenum, MoO₃, on carbon with a large specific surface area, and then by activating the molybdenum carbide formed by oxidation in a current of oxidising gas at a temperature of between 250 and 450°C.

4. A process for the isomerisation of hydrocarbons according to one of Claims 1 and 2, characterised in that the catalyst is prepared by carbonizing a paste composed of a mixture of an organic resin, molybdenum powder or a reducible compound of molybdenum, and by then heating the carbonised mixture to a temperature which is sufficient to reduce the molybdenum compound and to carbonize the molybdenum obtained, then by activating the molybdenum carbide formed by oxidation in a current of oxidising gas at a temperature of between 250 and 450°C.

5. A process for the isomerisation of hydrocarbons according to one of Claims 1 and 2, characterised in that the catalyst is obtained by immersing a mineral support into a suspension of Mo or of a reducible Mo compound in a carbonisable organic liquid, by carbonizing the suspension impregnating the support, by reducing the Mo compound, by carburising the molybdenum obtained, and finally by activating the molybdenum carbide formed by oxidation in a current of oxidising gas at a temperature of between 250 and 450°C.

6. A process for the isomerisation of hydrocarbons according to one of Claims 3 to 5, characterised in that the catalyst is subjected, prior to passage of the reaction mixture to be isomerized, to a treatment consisting in passing a mixture, at atmospheric pressure, of n-hexane and hydrogen over the activated catalyst at a temperature of between 250 and 450°C.

7. A process for the isomerisation of hydrocarbons according to one of Claims 1 and 2, characterised in that the catalyst is obtained by superficial oxidation of the metallic Mo into MoO₃ oxide and in that the superficial MoO₃ is partly carbonised by passage of the reaction mixture which itself contains hydrogen and the hydrocarbon(s) at a temperature of between 250 and 450°C.

8. A process for the isomerisation of hydrocarbons according to one of Claims 1 and 2, characterised in that the catalyst is obtained from MoO₃ which is partly carbonised by passage of the reaction mixture which itself contains hydrogen and the hydrocarbon(s) at a temperature of between 250 and 450°C.

9. A process for the isomerisation of hydrocarbons according to one of Claims 7 and 8, characterised in that before passing the reaction mixture over the molybdenum oxide MoO₃ the oxide is partly carbonised by passage of a mixture of n-hexane and hydrogen at a temperature of between 250 and 450°C.

10. A process for the isomerisation of hydrocarbons according to one of Claims 1 and 2, characterised in that the catalyst is obtained from a porous support of a product which slightly interacts with the molybdenum oxide MoO₃, coated with a layer of molybdenum oxide MoO₃, which is then partly carbonised by passage of a mixture of hydrocarbons and hydrogen at a temperature of between 250 and 450°C.

11. A process for the isomerisation of hydrocarbons according to Claim 10, characterised in that the support which slightly interacts with the molybdenum oxide belongs to the group of oxides TiO₂, SiO₂, and ZrO₂.

12. A process for the isomerisation of hydrocarbons according to Claim 10, characterised in that the support which slightly interacts with the molybdenum oxide is of silicon carbide SiC.

13. A process for the isomerisation of hydrocarbons according to one of Claims 10 to 12, characterised in that the porous support is covered with a layer of MoO₃ by impregnation with an aqueous solution of ammonium heptamolybdate of a quantity such that the content measured of metal molybdenum is between 5 and 16% of the weight of the support, dried for 8 to 20 hours at a temperature of between 100 and 150°C, then calcined in air at a temperature of between 400 and 600°C to transform the molybdate into MoO₃.

14. A process for the isomerisation of hydrocarbons according to one of Claims 10 to 13, characterised in that the mixture of hydrocarbons and hydrogen is the reaction mixture itself comprising the hydrocarbon(s) with more than 6 carbon atoms and hydrogen.

15. A process for the isomerisation of hydrocarbons according to one of Claims 10 to 13, characterised in that the mixture of hydrocarbon and hydrogen is initially a mixture of n-hexane and hydrogen and secondly the reaction mixture comprising the hydrocarbon(s) with at least 7 carbon atoms and hydrogen.

16. A process for the isomerisation of hydrocarbons according to one of Claims 1 to 15, characterised in that the hydrocarbon of the reaction mixture with at least 7 carbon atoms is n-heptane.

17. A process for the isomerisation of hydrocarbons according to one of Claims 1 to 15, characterised in that the reaction mixture contains hydrocarbons containing from 8 to 10 carbon atoms.

## Patentansprüche

1. Verfahren zur Isomerisierung von geradkettigen Kohlenwasserstoffen, die mindestens 7 Kohlenstoffatome enthalten,
**dadurch gekennzeichnet, daß**
ein Reaktionsgemisch, das den oder die zu isomerisierenden Kohlenwasserstoffe und Wasserstoff enthält, vorzugsweise bei einem Gesamtdruck von 100 bis 2000 kPa über einen Katalysator geleitet wird, der Molybdänverbindungen enthält, wobei zumindest die Oberfläche des Katalysators auf Molybdäncarbid besteht, das in Form eines oder mehrerer sauerstoffhaltiger Carbide teilweise oxidiert ist.

2. Verfahren zur Isomerisierung nach Anspruch 1, dadurch gekennzeichnet, daß das Reaktionsgemisch bei einer Temperatur im Bereich von 250 bis 450 °C in einer Konzentration des Kohlenwasserstoffs oder der Kohlenwasserstoffe in Wasserstoff im Bereich von 1 bis 70 Vol.-% über den Katalysator geleitet wird.

3. Verfahren zur Isomerisierung von Kohlenwasserstoffen nach einem der Ansprüche 1 oder 2, dadurch gekennzeichnet, daß der Katalysator hergestellt wird, indem eine flüchtige Molybdänverbindung MoO₃ mit Kohlenstoff von großer spezifischer Oberfläche umgesetzt und anschließend das gebildete Molybdäncarbid durch Oxidation, die unter einem oxidierenden Gasstrom bei einer Temperatur von 250 bis 450 °C durgeführt wird, aktiviert wird.

4. Verfahren zur Isomerisierung von Kohlenwasserstoffen nach einem der Ansprüche 1 oder 2, dadurch gekennzeichnet, daß der Katalysator durch Carbonisierung einer Paste, die aus einem Gemisch von organischem Harz und Molybdänpulver oder einer reduzierbaren Molybdänverbindung zusammengesetzt ist, anschließendes Erwärmen des carbonisierten Gemisches auf eine Temperatur, die ausreichend ist, um die Molybdänverbindung zu reduzieren und das erhaltene Molybdän in Carbid umzuwandeln, und anschließende Aktivierung des gebildeten Molybdäncarbids durch Oxidation, die unter einem oxidierenden Gastrom bei einer Temperatur im Bereich von 250 bis 450 °C durchgeführt wird, hergestellt wird.

5. Verfahren zur Isomerisierung von Kohlenwasserstoffen nach einem der Ansprüche 1 oder 2, dadurch gekennzeichnet, daß der Katalysator hergestellt wird, indem ein anorganischer Träger in eine Suspension von Mo oder einer reduzierbaren Mo-Verbindung in einer carbonisierbaren organischen Flüssigkeit eingetaucht, die den Träger imprägnierende Suspension carbonisiert, die Mo-Verbindung reduziert, das erhaltene Molybdän in Carbid umgewandelt, und schließlich das gebildete Molybdäncarbid durch Oxidation, die unter einem oxidierenden Gasstrom bei einer Temperatur von 250 bis 450 °C durchgeführt wird, aktiviert wird.

6. Verfahren zur Isomerisierung von Kohlenwasserstoffen nach einem der Ansprüche 3 bis 5, dadurch gekennzeichnet, daß der Katalysator, bevor das zu isomerisierende Reaktionsgemisch darübergeleitet wird, einer Behandlung unterzogen wird, die darin besteht, ein Gemisch von n-Hexan und Wasserstoff unter Atmosphärendruck bei einer Temperatur von 250 bis 450 °C über den aktivierten Katalysator zu leiten.

7. Verfahren zur Isomerisierung von Kohlenwasserstoffen nach einem der Ansprüche 1 oder 2, dadurch gekennzeichnet, daß der Katalysator hergestellt wird, indem das metallische Mo an der Oberfläche zu dem Oxid MoO₃ oxidiert wird, und dadurch, daß das an der Oberfläche vorhandene MoO₃ teilweise in Carbid umgewandelt wird, indem das Reaktionsgemisch selbst, das Wasserstoff und den Kohlenwasserstoff oder die Kohlenwasserstoffe enthält, bei einer Temperatur von 250 bis 450 °C darübergeleitet wird.

8. Verfahren zur Isomerisierung von Kohlenwasserstoffen nach einem der Ansprüche 1 oder 2, dadurch gekennzeichnet, daß der Katalysator aus MoO₃ hergestellt wird, das teilweise in Carbid umgewandelt wird, indem das Reaktionsgemisch selbst, das Wasserstoff und den Kohlenwasserstoff oder die Kohlenwasserstoffe enthält, bei einer Temperatur von 250 bis 450 °C darübergeleitet wird.

9. Verfahren zur Isomerisierung von Kohlenwasserstoffen nach einem der Ansprüche 7 oder 8, dadurch gekennzeichnet, daß, bevor das Reaktionsgemisch über das Molybdänoxid MoO₃ geleitet wird, das Oxid teilweise in Carbid umgewandelt wird, indem ein Gemisch von n-Hexan und Wasserstoff bei einer Temperatur von 250 bis 450 °C darübergeleitet wird.

10. Verfahren zur Isomerisierung von Kohlenwasserstoffen nach einem der Ansprüche 1 oder 2, dadurch gekennzeichnet, daß der Katalysator aus einem porösen Träger aus einem Produkt mit einer schwachen Wechselwirkung mit Molybdänoxid MoO₃ hergestellt wird, der mit einer Schicht von Molybdänoxid MoO₃ bedeckt ist, das anschließend teilweise in Carbid umgewandelt wird, indem ein Gemisch von Kohlenwasserstoffen und Wasserstoff bei einer Temperatur von 250 bis 450 °C darübergeleitet wird.

11. Verfahren zur Isomerisierung von Kohlenwasserstoffen nach Anspruch 10, dadurch gekennzeichnet, daß der Träger, der eine schwache Wechselwirkung mit Molybdänoxid aufweist, aus der Gruppe der Oxide TiO₂, SiO₂ und ZrO₂ stammt.

12. Verfahren zur Isomerisierung von Kohlenwasserstoffen nach Anspruch 10, dadurch gekennzeichnet, daß der Träger, der eine schwache Wechselwirkung mit Molybdänoxid aufweist, aus Siliciumcarbid SiC besteht.

13. Verfahren zur Isomerisierung von Kohlenwasserstoffen nach einem der Ansprüche 10 bis 12, dadurch gekennzeichnet, daß der poröse Träger mit einer MoO₃-Schicht überzogen wird, indem er mit einer wässerigen Lösung von Ammoniumheptamolybdat in einem Mengenanteil, der so ausgewählt ist, daß der Gehalt, bezogen auf metallisches Molybdän, im Bereich von 5 bis 16 Gew.-% des Trägers liegt, imprägniert, 8 bis 20 h bei einer Temperatur von 100 bis 150 °C getrocknet und anschließend an Luft bei einer Temperatur von 400 bis 600 °C calciniert wird, um das Molybdat in MoO₃ umzuwandeln.

14. Verfahren zur Isomerisierung von Kohlenwasserstoffen nach einem der Ansprüche 10 bis 13, dadurch gekennzeichnet, daß das Gemisch von Kohlenwasserstoffen und Wasserstoff das Reaktionsgemisch selbst ist, das den Kohlenwasserstoff oder die Kohlenwasserstoffe mit mehr als 6 Kohlenstoffatomen und Wasserstoff enthält.

15. Verfahren zur Isomerisierung von Kohlenwasserstoffen nach einem der Ansprüche 10 bis 13, dadurch gekennzeichnet, daß das Gemisch von Kohlenwasserstoffen und Wasserstoffen in einem ersten Schritt ein Gemisch von n-Hexan und Wasserstoff und in einem zweiten Schritt das Reaktionsgemisch ist, das den Kohlenwasserstoff oder die Kohlenwasserstoffe mit mindestens 7 Kohlenstoffatomen und Wasserstoff enthält.

16. Verfahren zur Isomerisierung von Kohlenwasserstoffen nach einem der Ansprüche 1 bis 15, dadurch gekennzeichnet, daß der Kohlenwasserstoff des Reaktionsgemisches, der mindestens 7 Kohlenstoffatome aufweist, n-Heptan ist.

17. Verfahren zur Isomerisierung von Kohlenwasserstoffen nach einem der Ansprüche 1 bis 15, dadurch gekennzeichnet, daß das Reaktionsgemisch Kohlenwasserstoffe mit 8 bis 10 Kohlenstoffatomen enthält.
